(19) Europäisches Patentamt / European Patent Office / Office européen des brevets

(11) **EP 4 122 327 A1**

(12) **EUROPEAN PATENT APPLICATION**
published in accordance with Art. 153(4) EPC

(43) Date of publication:
**25.01.2023 Bulletin 2023/04**

(21) Application number: **21771002.9**

(22) Date of filing: **17.03.2021**

(51) International Patent Classification (IPC):
**A23L 5/00** *(2016.01)*    **C12N 9/02** *(2006.01)*
**C12N 9/78** *(2006.01)*    **C12P 21/00** *(2006.01)*

(52) Cooperative Patent Classification (CPC):
**A23L 5/00; C12N 9/0004; C12N 9/78; C12P 21/00**

(86) International application number:
**PCT/JP2021/010764**

(87) International publication number:
**WO 2021/187510 (23.09.2021 Gazette 2021/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2020 JP 2020046931**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **PROTEIN CROSSLINKING METHOD**

(57) The present invention addresses the problem of providing a novel protein crosslinking method. In the present invention, a crosslinking reaction is accelerated by causing both an oxidoreductase such as a laccase and a protein deamidase such as a protein glutaminase to act on a substrate protein.

EP 4 122 327 A1

**Description**

TECHNICAL FIELD

The present invention relates to a novel protein crosslinking method using enzymes. Specifically, the present invention relates to a protein crosslinking method using an oxidoreductase and a protein deamidase in combination.

BACKGROUND ART

As an enzyme having a possibility of polymerizing proteins by a crosslinking reaction, transglutaminase, lysyl oxidase, protein disulfide isomerase, protein disulfide reductase, sulfhydryl oxidase, lipoxygenase, polyphenol oxidase (tyrosinase), peroxidase, and the like have been conventionally known (see, for example, Non-Patent Document 1).

Regarding transglutaminase among the above-described enzymes, a protein crosslinking method using the transglutaminase is well known. It is well known that owing to the discovery of transglutaminase that is derived from microorganisms, is inexpensive, and does not require the presence of calcium for reaction, the transglutaminase is widely used mainly in the food processing field (see Patent Document 1 and Non-Patent Document 2).

However, the protein crosslinking reaction by transglutaminase has the following problems. That is, since transglutaminase is an enzyme that forms a crosslinking structure in a protein molecule or between protein molecules as a result of an acyl transition reaction that occurs between a $\gamma$-carboxyl group of a glutamine residue and an $\varepsilon$-amino group of a lysine residue in a protein, there are proteins that are difficult to serve as a substrate due to a shortage of a glutamine residue or a lysine residue depending on the type of proteins. For example, albumin proteins are incapable of serving as a substrate for transglutaminase in a native state.

As described above, conventionally, as a protein crosslinking method by an enzyme, the possibility of using some enzymes has been pointed out, but there is almost no practical method satisfying the supply amount, cost, ease of purification, and the like. Even in the only method using a microorganism-derived transglutaminase, a crosslinking reaction does not occur depending on the type of protein, and the use thereof has been limited.

As a countermeasure, a protein crosslinking method by multicopper oxidase including laccase, bilirubin oxidase, ascorbic acid oxidase, ceruloplasmin, and the like having a completely different reaction mechanism from that of transglutaminase has been proposed, and a range of target proteins that has been limited in the protein crosslinking method by transglutaminase is expanded, and a method for producing a protein gelled substance having novel physical properties and nature has become possible (see Patent Document 2).

PRIOR ART DOCUMENTS

PATENT DOCUMENTS

    Patent Document 1: JP H06-65280 A
    Patent Document 2: JP Japanese Patent Laid-open Publication No. H11-276162

NON-PATENT DOCUMENTS

    Non-Patent Document 1: Matheis and Whitaker, J. Food Biochemistry 11, 309-327, 1987
    Non-Patent Document 2: Nippon Nogeikagaku Kaishi, vol. 69, No. 10, pp. 1301-1308

SUMMARY OF THE INVENTION

PROBLEMS TO BE SOLVED BY THE INVENTION

However, in the protein crosslinking method by multicopper oxidase as described above, since the reactivity of multicopper oxidase with proteins is low, there are problems that a large amount of enzyme is required and a long reaction time is required, for example.

EP 4 122 327 A1

MEANS FOR SOLVING THE PROBLEMS

As a result of intensive studies to solve the above problems, the present inventor has newly found that a crosslinking reaction of a protein by an oxidoreductase such as multicopper oxidase is significantly improved by using enzymes that deamidate a protein in combination, and has completed the present invention described below.

[1] A protein crosslinking method characterized by allowing an oxidoreductase and a protein deamidase to act on a protein.
[2] The protein crosslinking method according to [1], wherein the oxidoreductase is multicopper oxidase.
[3] The protein crosslinking method according to [2], wherein the multicopper oxidase is laccase and/or bilirubin oxidase.
[4] The protein crosslinking method according to [2], wherein the multicopper oxidase is laccase.
[5] The protein crosslinking method according to any one of [1] to [4], wherein the protein deamidase is an enzyme that acts on a glutamine residue in a protein.
[6] The protein crosslinking method according to [5], wherein the protein deamidase is protein glutaminase.
[7] A protein improving agent containing:

an oxidoreductase; and
a protein deamidase.

[8] The protein improving agent according to [7], wherein the oxidoreductase is multicopper oxidase.
[9] The protein improving agent according to [8], wherein the multicopper oxidase is laccase and/or bilirubin oxidase.
[10] The protein improving agent according to [8], wherein the multicopper oxidase is laccase.
[11] The protein improving agent according to any one of [7] to [10], wherein the protein deamidase is an enzyme that acts on a glutamine residue in a protein.
[12] The protein improving agent according to [11], wherein the protein deamidase is protein glutaminase.
[13] A method for producing a crosslinked protein, the method including the steps of:

(1) treating a protein with a protein deamidase; and
(2) treating the protein subjected to protein deamidation with an oxidoreductase.

[14] A method for producing a crosslinked protein, the method including the steps of:

(1) preparing a protein treated with a protein deamidase; and
(2) treating the prepared protein with an oxidoreductase.

[15] A method for producing a crosslinked protein, the method including simultaneously treating a protein with an oxidoreductase and a protein deamidase.
[16] A method for producing a food or a pharmaceutical product, the method including the steps of:

(1) preparing a food raw material or pharmaceutical raw material containing a protein and treated with a protein deamidase; and
(2) treating the prepared food raw material or pharmaceutical raw material with an oxidoreductase.

[17] A method for producing a food or a pharmaceutical product, the method including a step of simultaneously treating a food raw material or a pharmaceutical raw material containing a protein with an oxidoreductase and a protein deamidase.

BRIEF DESCRIPTION OF THE DRAWINGS

Fig. 1 is a diagram illustrating an electrophoresis pattern of SDS-polyacrylamide gel electrophoresis in Test Example 1.
Fig. 2 is a diagram illustrating an electrophoresis pattern of SDS-polyacrylamide gel electrophoresis in Test Example 2.
Fig. 3 is a diagram illustrating an electrophoresis pattern of SDS-polyacrylamide gel electrophoresis in Test Example 3.

Fig. 4 is a diagram illustrating an electrophoresis pattern of SDS-polyacrylamide gel electrophoresis in Test Example 5.

Fig. 5 is a diagram illustrating an electrophoresis pattern of SDS-polyacrylamide gel electrophoresis in Test Example 6.

Fig. 6 is a diagram illustrating an electrophoresis pattern of SDS-polyacrylamide gel electrophoresis in Test Example 7.

Fig. 7 is a diagram illustrating an electrophoresis pattern of SDS-polyacrylamide gel electrophoresis in Test Example 8.

Fig. 8 is a graph illustrating viscosity changes in Test Example 9.

EMBODIMENTS OF THE INVENTION

1. Protein Crosslinking Method

A crosslinking method of the present invention includes allowing an oxidoreductase and a protein deamidase to act on a protein. The oxidoreductase referred to in the present invention is not particularly limited as long as it is an enzyme that crosslinks proteins by an oxidation-reduction reaction, and examples thereof include the following.

(1) Enzymes that crosslink proteins by oxidizing an $\varepsilon$-amino group of lysine in the proteins to produce aldehyde with high reactivity, and forming a Schiff base with the amino group of another protein molecule (for example, lysyl oxidase).
(2) Enzymes that crosslink proteins by oxidizing a sulfhydryl group of cysteine in the proteins and forming a disulfhydryl bond with another protein molecule (for example, sulfhydryl oxidase).
(3) Enzymes that crosslink proteins by oxidizing hydroxyl groups of tyrosine in the proteins to produce o-quinone with high reactivity, and reacting with quinone, an amino group, or a sulfhydryl group of another protein molecule (for example, tyrosinase).
(4) Enzymes that have wide substrate specificity, act mainly on a hydroxyl group of tyrosine in a protein, a sulfhydryl group of cysteine, or an $\varepsilon$-amino group of lysine, and crosslink proteins by any of the mechanisms (1) to (3) (for example, multicopper oxidase including laccase).
(5) Enzymes that catalyze the same reaction as (4) but require hydrogen peroxide as an oxygen donor in the oxidation reaction (for example, peroxidase).

Here, the multicopper oxidase is a group of enzymes that contain a plurality of copper atoms in the molecule and oxidize polyphenol, methoxyphenol, diamine, bilirubin, ascorbic acid, and the like with molecular oxygen. The number of contained copper atoms is usually 2 to 8 as known so far, but the number is not particularly limited because the number varies depending on the state of the enzyme preparation at the time of analysis and the analysis method. Examples of the enzyme classified as the multicopper oxidase include laccase, bilirubin oxidase, ascorbic acid oxidase, and ceruloplasmin.

Laccase ([EC 1.10.3.2]) is one kind of multicopper protein, is an enzyme having low substrate specificity, and also acts on o-quinol, p-quinol, or often aminophenol or phenylenediamine. The produced semiquinones react further enzymatically or nonenzymatically. Examples of such laccase include those derived from plants such as lacquer and microorganisms such as bacteria and fungi, and examples of the laccase derived from microorganisms include enzymes derived from the genus Aspergillus, Neurospora, Podospora, Botrytis, Collybia, Fomes, Lentinus, Pleurotus, Pycnoporus, Pyricularia, Trametes, Rhizoctonia, Rigidoporus, Coprinus, Psatyrella, Myceliophtera, Schtalidium, Polyporus, Phlebia, and Coriolus.

Bilirubin oxidase (EC 1.3.3.5) is one kind of multicopper protein, is an enzyme that mainly acts on bilirubin, and examples of such bilirubin oxidase include enzymes derived from the genus Penicillium, Myrothecium, and Trachyderma.

Ascorbic acid oxidase (EC 1.10.3.3) is one kind of multicopper protein, is an enzyme that mainly acts on L-ascorbic acid, and includes enzymes derived from plants such as cucumber, squash, and zucchini, and microorganisms such as bacteria and fungi.

Ceruloplasmin (EC 1.16.3.1) is one kind of multicopper protein, is a multifunctional protein having homeostasis maintenance of copper in a living body, ferroxidase activity, and amine oxidase activity, and is present in the serum of animals and birds.

The protein deamidase referred to in the present invention refers to an enzyme that catalyzes a reaction of liberating ammonia from proteins, and specific examples thereof include an enzyme that converts a glutamine residue in a protein into a glutamic acid residue (that is, protein glutaminase), an enzyme that converts an asparagine residue into an aspartic acid residue (that is, protein asparaginase), and a protein deiminase that converts an arginine residue in a protein into a citrulline residue. As an enzyme that deamidates a glutamine residue in a protein, for example, protein glutaminase derived from Chryseobacterium proteolyticum (EurJBiochem, 268 (5), 1410, 2001, Protein-glutaminase From Chryseobacterium Proteolyticum, an Enzyme That Deamidates Glutaminyl Residues in Proteins. Purification, Characterization and Gene Cloning, S Yamaguchi 1, DJ Jeenes, DBArcher or Front Microbiol, 9, 1975, 2018, Complete Genome Sequence and Characterization of a Protein-Glutaminase Producing Strain, Chryseobacterium proteolyticum QSH1265, Ruidan Qu, Xiaoyu Zhu, Min Tian, Yingjie Liu, Wenjuan Yan, Jian Ye, Hongliang Gao, Jing Huang) is well known, but the enzyme is not limited thereto. Examples of the enzyme that deamidates an asparagine residue in a protein include protein asparaginase disclosed in WO 2015/133590, but are not limited thereto. As an enzyme that deiminates arginine residues in a protein, for example, arginine deiminase derived from Fusarium graminearum is known.

In general, when a glutamine residue and/or an asparagine residue in a protein are deamidated to produce a carboxyl group, and the negative charge of the protein increases, and/or when an arginine residue having strong basicity in the protein is deiminated and neutralized, the isoelectric point decreases and the hydration force increases as a result thereof. Furthermore, an increase in electrostatic repulsion leads to a decrease in interaction between proteins, that is, a decrease in associative properties. These changes greatly increase the solubility and water dispersibility of the protein. Further, the increase in the negative charge of the protein unfolds folding of the protein, changes the higher-order structure, and exposes the hydrophobic region buried inside the molecule to the molecular surface. Therefore, the deamidated protein has amphiphilicity and serves as an ideal surfactant, and the emulsifying power, emulsion stability, foaming property, and foam stability of the protein are greatly improved. As described above, deamidation of a protein leads to improvement of various functional properties of the protein, and the use of the protein is exponentially increased (for example, Molecular Approaches to Improving Food Quality and Safety, D. Chatnagar and T. E .Cleveland, eds., Van Nostrand Reinhold, New York, 1992, p. 37). Further, even when an arginine residue in a protein is deiminated, the hydrophobicity of the protein is increased to change the higher-order structure of the protein.

Therefore, although not bound by theory, "a phenomenon in which the crosslinking reaction of a protein by an oxidoreductase is promoted by the action of a protein deamidase" (see Examples described later) newly found by the present inventor can be explained as follows. The action of the protein deamidase unfolds folding of the protein, and the higher-order structure is changed. As a result, amino acid residues targeted by the oxidoreductase, such as cysteine and lysine, including tyrosine buried inside the protein molecule, are exposed on the molecular surface of the protein, whereby the reaction is easily subjected to the action of the oxidoreductase.

Next, the protein crosslinking method in the present invention will be described in more detail. The type, origin, and the like of the oxidoreductase and the protein deamidase that can be used in the present invention are not particularly limited. The origin is an animal, a plant, or a microorganism, for example. Further, in the case of the enzyme derived from a microorganism, the enzyme may be accumulated in either inside or outside the cell of the microorganism. Furthermore, the enzyme may be not only a naturally existing enzyme but also an enzyme produced by genetic engineering or cell engineering techniques. Further, the enzymes may be an enzyme protein modified by a protein engineering technique. Furthermore, as the oxidoreductase (for example, multicopper oxidase) and the protein deamidase (for example, protein glutaminase), it is desirable to use purified enzymes with high purity, but the purity is not limited as long as a desired reaction is possible. Further, an enzyme preparation may be used as the oxidoreductase and the protein deamidase, and in this case, various salts, saccharides, proteins, lipids, surfactants, or the like as an enzyme stabilizer may be added to the enzyme preparation.

The crosslinking method of the present invention can be applied to various proteins for which crosslinking is desired. There are no particular restrictions on the origin, properties, or the like of the oxidoreductase and the protein that is a substrate of the protein deamidase. Examples of the vegetable protein include proteins derived from beans such as soybeans, green peas, lentils, chickpeas, and black beans, proteins derived from cereals such as wheat, barley, oat, and rice, proteins derived from nuts such as almonds and peanuts, and proteins derived from seeds such as hemp seed, chia seed, quinoa, and amaranthus. Further, insect proteins such as those of crickets, proteins derived from fungi such as yeast and filamentous fungi, and mycoproteins of mushrooms, and proteins derived from algae such as spirulina can also be used. Examples of the animal protein include milk proteins such as casein and β-lactoglobulin, egg proteins such as ovalbumin, meat proteins such as myosin and actin, blood proteins such as serum albumin, and tendon proteins such as gelatin and collagen. Further, a protein chemically partially decomposed by an acid, an alkali, or the like, a protein enzymatically partially decomposed by a protease or the like, a protein chemically modified by various reagents, a synthetic peptide, or the like can also be used as the substrate protein.

The substrate protein as described above is subjected to the reaction in a state of being contained in a fluid composition such as a solution, a slurry, or a paste. The concentration of the substrate protein in the fluid composition is not particularly limited, and the concentration only needs to be determined according to the desired property and state of the target protein crosslinked product. In general, a solution or precipitate with increased viscosity is obtained at a low concentration, and a gel-like substance is obtained at a high concentration, but a gelled substance can be sufficiently obtained when the substrate protein concentration is greater than or equal to 1% by weight. Further, the fluid composition containing a substrate protein is not limited to a fluid composition in the form of an aqueous solution, an aqueous dispersion, or an aqueous dispersion paste of a protein, and a fluid composition in the form in which these constitute an emulsion with an oil and fat may be subjected to a reaction, and salts, saccharides, proteins, flavors, humectants, coloring agents, and the like may be added to the fluid composition containing a substrate protein if necessary.

The amount of the enzyme used, reaction time, temperature, pH of the reaction solution, and the like are not particularly limited. Usually, the amount of the enzyme is 1 to 1,000,000 U, preferably 10 to 500,000 U, and more preferably 100 to 200,000 U of the oxidoreductase, and the amount of the protein deamidase is 0.01 to 100,000 U, preferably 0.1 to 50,000 U, and more preferably 1 to 10,000 U per 1 g of the protein. The reaction temperature is 5 to 80°C, preferably 20 to 60°C. The pH of the reaction solution is 2 to 10, preferably 4 to 8. The reaction time is 10 seconds to 48 hours, preferably 10 minutes to 24 hours. Under the above reaction conditions, it is possible to obtain a crosslinked product in which the protein is polymerized or a gel-like substance of a fluid composition. These reaction conditions are appropriately selected according to the physical properties and moisture content of the intended protein crosslinked product or gel-like substance of the fluid composition. Note that the optimum reaction conditions are preferably determined through a preliminary experiment.

When multicopper oxidase is used as the oxidoreductase, various polyphenols such as hydroquinone, catechol, guaiacol, ferulic acid, vanillic acid, p-coumaric acid, syringaldehyde, and p-phenylenediamine may be added as a mediator for promoting the reaction.

In the protein crosslinking method in the present invention, a substrate protein is treated with enzymes for protein crosslinking, that is, an oxidoreductase and a protein deamidase. The order in which the enzymes are allowed to act (that is, the order of the treatment with an oxidoreductase and the treatment with a protein deamidase) is not particularly limited, but it is preferable that the substrate protein is treated simultaneously with both the enzymes or treated with a protein deamidase and then treated with an oxidoreductase. The simultaneous treatment is more preferable for the purpose of improving work efficiency, for example. When the substrate protein is treated with the protein deamidase and then treated with the oxidoreductase, a step of deactivating the protein deamidase may be added after the protein deamidase treatment. It is possible to adjust the amount of protein deamidation by adding the deactivation step.

2. Method for Producing Crosslinked Protein, or Food and Pharmaceutical Product Containing Crosslinked Protein

By using the crosslinking method of the present invention, a crosslinked protein, or a food or a pharmaceutical product containing the crosslinked protein can be produced. One aspect of the method for producing a crosslinked protein includes the following steps (1) and (2). Note that the deactivation step of a protein deamidase may be added after the step (1).

(1) a step of treating a protein with a protein deamidase
(2) a step of treating the deamidated protein with an oxidoreductase

In another aspect, the following steps (1) and (2) are performed.

(1) a step of preparing a protein treated with a protein deamidase
(2) a step of treating the prepared protein with an oxidoreductase

In still another aspect, a crosslinked protein is produced by the following step (i).

(i) a step of simultaneously treating a protein with an oxidoreductase and a protein deamidase

Meanwhile, one aspect of a method for producing a food or medicine includes the following (1) and (2).

(1) a step of preparing a food raw material or pharmaceutical raw material containing a protein and treated with a protein deamidase
(2) a step of treating the prepared food raw material or pharmaceutical raw material with an oxidoreductase

In another aspect, a food or a pharmaceutical product is produced by the following step (i).

(i) a step of simultaneously treating a food raw material or a pharmaceutical raw material containing a protein with an oxidoreductase and a protein deamidase.

3. Protein Improving Agent

The present invention also provides a protein improving agent that can be used for crosslinking proteins. The protein improving agent of the present invention is typically used in the crosslinking method or the production method of the present invention. The protein improving agent of the present invention contains an oxidoreductase and a protein deamidase, which are enzymes for crosslinking proteins, as active ingredients. The protein improving agent of the present invention can be used as a protein crosslinking agent, and can be preferably used as a thickener for a fluid composition containing a protein, and more preferably used as a gelling agent for a fluid composition containing a protein. Since the details of the oxidoreductase and the protein deamidase are as described above (the column of 1. Protein Crosslinking Method), the description thereof is omitted.

Hereinafter, the present invention will be further described with reference to examples.

EXAMPLES

In the following examples, the enzyme activity of laccase was measured by the method described below using 2,2'-azino-di-[3-ethylbenzthiazolinesulfonate(6)] (ABTS, manufactured by Boehringer Mannheim) as a substrate unless otherwise specified.

<Activity Measurement Method>

ABTS is dissolved in a 25 mM citrate buffer solution (pH 3.2) at a concentration of 1.0 mg/ml to prepare a substrate solution. After 3.0 ml of the substrate solution is taken into a cuvette and preheated at 25°C, 0.1 ml of an enzyme solution is added, the mixture is stirred and incubated at 25°C, and the absorbance of the mixture at 405 nm after 1 minute and 3 minutes is measured. The amount of the enzyme that increases the absorbance at 405 nm by 1.0 OD per minute under these conditions is defined as 1 unit.

Meanwhile, the enzyme activity of protein glutaminase was measured by the method described below using Z-Gln-Gly as a substrate unless otherwise specified.

<Activity Measurement Method>

After 10 $\mu$l of an enzyme solution is added to 100 $\mu$l of a 176 mmol/l phosphate buffer solution (pH 6.5) containing 10 mmol/l Z-Gln-Gly and the mixture is incubated at 37°C for 60 minutes, 100 $\mu$l of a 12% trichloroacetic acid solution is added to stop the reaction. After centrifugation (15,000 rpm, 4°C, 5 min), the supernatant is subjected to measurement using F-kit ammonia (manufactured by Boehringer Mannheim) as follows (A1). Separately, measurement is performed in the same manner using water instead of the enzyme solution (A2). After 10 $\mu$l of the supernatant and 190 $\mu$l of water are added to 100 $\mu$l of F-kit ammonia reagent 2 and the mixture is allowed to stand at room temperature for 5 minutes, the absorbance (E1) at 340 nm is measured using 100 $\mu$l of the mixture. After 1.0 $\mu$l of reagent 3 (glutamate dehydrogenase) is added to the remaining 200 $\mu$l of the mixture, the mixture is further allowed to stand at room temperature for 20 minutes, and then the absorbance (E2) of the remaining 200 $\mu$l of the mixture at 340 nm is measured. The amount of the enzyme that liberates 1 $\mu$mol of ammonia per minute under the above conditions is defined as 1 unit, and is determined according to the following equation.

$$u/ml = 1.76 \times [A1(E1 - E2) - A2(E1 - E2)]$$

<Test Example 1>

The effect of promoting protein crosslinking by using laccase (LC) in combination with protein glutaminase (PG) was examined by a method using egg-derived albumin (manufactured by FUJIFILM Wako Pure Chemical Corporation). Egg-derived albumin in an amount of 5% by weight (final concentration), a 50 mM (final concentration) potassium sodium phosphate buffer solution (pH 7.0), laccase (product name: Laccase Y120, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where laccase was used alone. The enzyme addition amount was 100 U of laccase (final concentration) and 500 mU of protein glutaminase (final concentration) per 1 mg of the substrate protein.

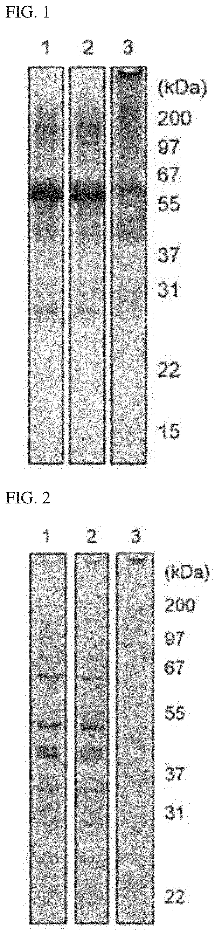

The results are shown in Fig. 1 and Table 1.

[Table 1]

| Lane | Added enzyme | Crosslinking | Crosslink promotion effect |
|---|---|---|---|
| 1 | No added enzyme | No | |

(continued)

| Lane | Added enzyme | Crosslinking | Crosslink promotion effect |
|------|-------------|-------------|----------------------------|
| 2 | LC | No | |
| 3 | LC + PG | Yes | Yes |

As shown in Fig. 1, when laccase was used alone, crosslinking polymerization of the substrate protein did not occur. Meanwhile, when laccase and protein glutaminase were used in combination, a band of a protein that was so highly polymerized that the protein was incapable of passing through the mesh of the polyacrylamide gel was observed at the top part of lane 3, and crosslinking polymerization of the substrate protein was observed. That is, by using laccase in combination with protein glutaminase, the effect of promoting crosslinking of egg-derived albumin proteins was observed.

<Test Example 2>

The effect of promoting protein crosslinking by using laccase in combination with protein glutaminase was examined using LYZAMINE-S (pea protein, manufactured by Roquette Japan K.K.). LYZAMINE-S in an amount of 5% by weight (final concentration), a 50 mM (final concentration) potassium sodium phosphate buffer solution (pH 7.0), laccase (product name: Laccase Y120, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where laccase was used alone. The enzyme addition amount was 100 U of laccase (final concentration) and 500 mU of protein glutaminase (final concentration) per 1 mg of the substrate protein.
The results are shown in Fig. 2 and Table 2.

[Table 2]

| Lane | Added enzyme | Crosslinking | Crosslink promotion effect |
|------|-------------|-------------|----------------------------|
| 1 | No added enzyme | No | |
| 2 | LC | No | |
| 3 | LC + PG | Yes | Yes |

As shown in Fig. 2, when laccase was used alone, crosslinking polymerization of the substrate protein did not occur. Meanwhile, when laccase and protein glutaminase were used in combination, a band of a protein that was so highly polymerized that the protein was incapable of passing through the mesh of the polyacrylamide gel (a band observed at the top part of lane 3) was observed to be denser, and crosslinking polymerization of the substrate protein was observed. That is, by using laccase in combination with protein glutaminase, the effect of promoting crosslinking of pea proteins was observed.

<Test Example 3>

The effect of promoting protein crosslinking by using laccase in combination with protein glutaminase was examined by a method using a soybean-derived protein powder (manufactured by FUJIFILM Wako Pure Chemical Corporation). The soybean-derived protein powder in an amount of 5% by weight (final concentration), a 50 mM (final concentration) potassium sodium phosphate buffer solution (pH 7.0), laccase (product name: Laccase Y120, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where the same concentration of laccase was used alone. The enzyme addition amount was 100 U of laccase (final concentration) and 500 mU of protein glutaminase (final concentration) per 1 mg of the substrate protein. Further, the same reaction was also performed under the conditions where the enzyme addition amounts (laccase and protein glutaminase) were reduced to 1/10 and 1/100, respectively.

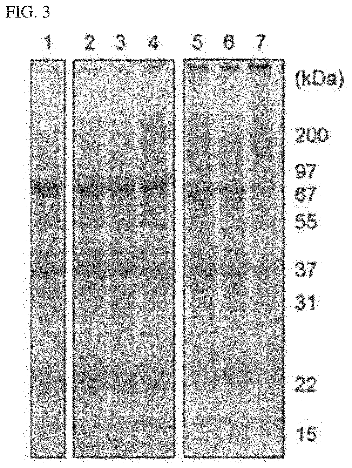

The results are shown in Fig. 3 and Table 3.

[Table 3]

| Lane | Added enzyme | Crosslinking | Crosslink promotion effect |
|---|---|---|---|
| 1 | No added enzyme | No | |
| 2 | LC (1/100 amount) | No | |
| 3 | LC (1/10 amount) | No | |
| 4 | LC | Yes | |
| 5 | LC + PG (1/100 amount) | Yes | Yes |
| 6 | LC + PG (1/10 amount) | Yes | Yes |
| 7 | LC + PG | Yes | Yes |

As shown in Fig. 3, when laccase and protein glutaminase were used in combination, the efficiency of crosslinking polymerization of the substrate protein was improved as compared with the case where laccase was used alone, and at any enzyme concentration, a band of the protein that was so highly polymerized that the protein was incapable of passing through the mesh of the polyacrylamide gel (a band that can be observed at the top part of each lane (lanes 5 to 7)) was observed to be denser, and crosslinking polymerization was observed. That is, by using laccase in combination with protein glutaminase, the effect of promoting crosslinking of soybean-derived proteins was observed.

<Test Example 4>

The effect of promoting protein crosslinking by using laccase in combination with protein glutaminase was examined by a method using egg-derived albumin (manufactured by FUJIFILM Wako Pure Chemical Corporation). Egg-derived albumin in an amount of 5% by weight (final concentration), a 50 mM (final concentration) potassium sodium phosphate buffer solution (pH 7.0), and protein glutaminase (product name: Protein Glutaminase "Amano" 500, manufactured by Amano Enzyme Inc.) were mixed, and then the mixture was performed at 40°C for 4 hours with being shaken at 160 rpm. Thereafter, laccase (product name: Laccase Y120, manufactured by Amano Enzyme Inc.) was added, and the mixture was reacted at 40°C for 20 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking. The enzyme addition amount was 100 U of laccase (final concentration) and 500 mU of protein glutaminase (final concentration) per 1 mg of the substrate protein.

The results are shown in Table 4.

[Table 4]

| Lane | Added enzyme | Crosslinking |
|---|---|---|
| 1 | No added enzyme | No |
| 2 | PG → LC | Yes |

Also in the electrophoretic image, even when protein glutaminase treatment was followed by laccase treatment, a band of the protein that was so highly polymerized that the protein was incapable of passing through the mesh of the polyacrylamide gel was observed at the top part of lane 2, and crosslinking polymerization of the substrate protein was observed. As is clear from the comparison between the case where the treatment was performed using laccase alone in Test Example 1 and the case where the laccase treatment was performed after the protein glutaminase treatment in this test example, by using protein glutaminase in combination with the laccase, the effect of promoting crosslinking of egg-derived albumin proteins was observed regardless of the order of addition of these enzymes.

<Test Example 5>

The effect of promoting protein crosslinking by using laccase in combination with protein glutaminase was examined by a method using an almond-derived protein powder. The almond-derived protein powder in an amount of 5% by weight (final concentration), a 50 mM (final concentration) potassium sodium phosphate buffer solution (pH 7.0), laccase (product name: Laccase Y120, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where the same concentration of laccase was used alone. The enzyme addition amount was 100 U of laccase (final concentration) and 500 mU of protein glutaminase (final concentration) per 1 mg of the substrate protein. Further, the same reaction was also performed under the conditions where both the enzyme addition amounts (laccase and protein glutaminase) were reduced.

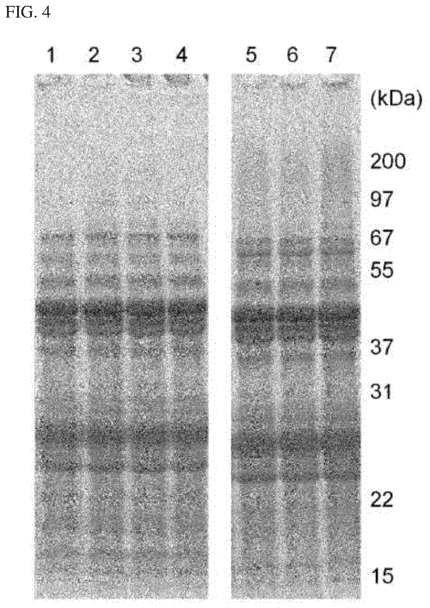

The results are shown in Fig. 4 and Table 5.

[Table 5]

| Lane | Added enzyme | Crosslinking | Crosslink promotion effect |
|------|--------------|--------------|----------------------------|
| 1 | No added enzyme | No | |
| 2 | LC (1/100 amount) | No | |
| 3 | LC (1/10 amount) | No | |
| 4 | LC | No | |
| 5 | LC + PG (1/100 amount) | Yes | Yes |
| 6 | LC + PG (1/10 amount) | Yes | Yes |
| 7 | LC + PG | Yes | Yes |

As shown in Fig. 4, when laccase and protein glutaminase were used in combination, the efficiency of crosslinking and polymerizing the substrate protein was improved as compared with the case where laccase was used alone, and at any enzyme concentration, the presence of a protein polymerized to greater than or equal to about 200 kDa was newly confirmed, and crosslinking polymerization was observed (lanes 5 to 7). That is, by using laccase in combination with protein glutaminase, the effect of promoting crosslinking of almond-derived proteins was observed.

<Test Example 6>

The effect of promoting protein crosslinking by using laccase in combination with protein glutaminase was examined by a method using a chickpea-derived protein powder. The chickpea-derived protein powder in an amount of 5% by weight (final concentration), a 50 mM (final concentration) potassium sodium phosphate buffer solution (pH 7.0), laccase (product name: Laccase Y120, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where the same concentration of laccase was used alone. The enzyme addition amount was 100 U of laccase (final concentration) and 500 mU of protein glutaminase (final concentration) per 1 mg of the substrate protein. Further, the same reaction was also performed under the conditions where both the enzyme addition amounts (laccase and protein glutaminase) were reduced.

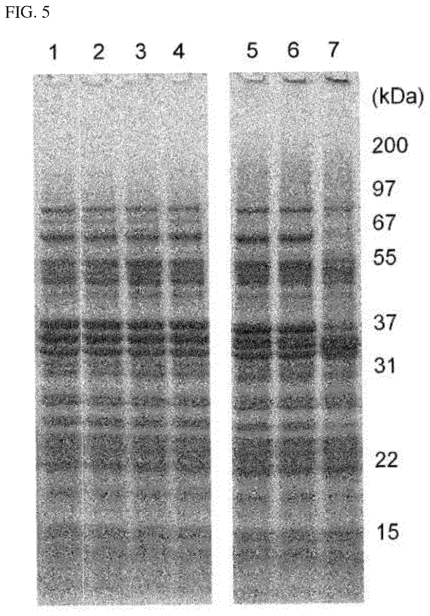

The results are shown in Fig. 5 and Table 6.

[Table 6]

| Lane | Added enzyme | Crosslinking | Crosslink promotion effect |
|------|--------------|--------------|----------------------------|
| 1 | No added enzyme | No | |
| 2 | LC (1/100 amount) | No | |
| 3 | LC (1/10 amount) | No | |
| 4 | LC | No | |
| 5 | LC + PG (1/100 amount) | Yes | Yes |
| 6 | LC + PG (1/10 amount) | Yes | Yes |
| 7 | LC + PG | Yes | Yes |

As shown in Fig. 5, when laccase and protein glutaminase were used in combination, the efficiency of crosslinking and polymerizing the substrate protein was improved as compared with the case where laccase was used alone, and at any enzyme concentration, a band of the protein that was so highly polymerized that the protein was incapable of passing through the mesh of the polyacrylamide gel (a band that can be observed at the top part of each lane (lanes 5 to 7)) was observed to be denser, and crosslinking polymerization was observed. That is, by using laccase in combination with protein glutaminase, the effect of promoting crosslinking of chickpea-derived proteins was observed.

<Test Example 7>

The effect of promoting protein crosslinking by using bilirubin oxidase (BO) in combination with protein glutaminase was examined by a method using casein (Merck Millipore). The protein in an amount of 5% by weight (final concentration), a 50 mM (final concentration) potassium sodium phosphate buffer solution (pH 7.0), bilirubin oxidase (product name: BO "Amano" 3, manufactured by Amano Enzyme Inc.) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and the crosslinking promotion effect relative to the case where the same concentration of bilirubin oxidase was used alone. The enzyme addition amount was 100 U of bilirubin oxidase (final concentration) and 500 mU of protein glutaminase (final concentration) per 1 mg of the substrate protein. Further, the same reaction was also performed under the conditions where both the enzyme addition amounts (bilirubin oxidase and protein glutaminase) were reduced.

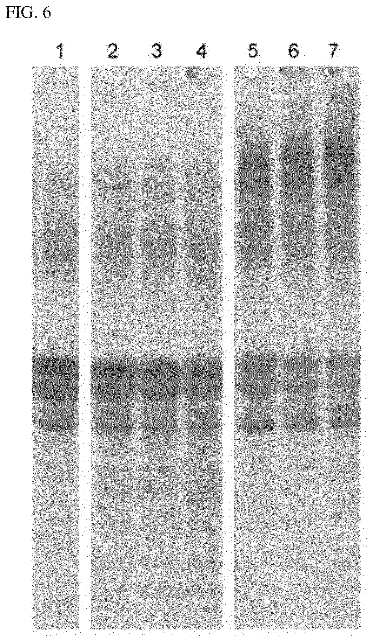

The results are shown in Fig. 6 and Table 7.

[Table 7]

| Lane | Added enzyme | Crosslinking | Crosslink promotion effect |
|---|---|---|---|
| 1 | No added enzyme | No | |
| 2 | BO (1/100 amount) | No | |
| 3 | BO (1/10 amount) | No | |
| 4 | BO | No | |
| 5 | BO + PG (1/100 amount) | Yes | Yes |
| 6 | BO + PG (1/10 amount) | Yes | Yes |
| 7 | BO + PG | Yes | Yes |

As shown in Fig. 6, when bilirubin oxidase and protein glutaminase were used in combination, the efficiency of crosslinking polymerization of the substrate protein was improved as compared with the case where bilirubin oxidase was used alone, and at any enzyme concentration, a band of the polymerized protein was observed to be denser at the upper part of each lane (lanes 5 to 7), and crosslinking polymerization was observed. That is, by using bilirubin oxidase in combination with protein glutaminase, the effect of promoting crosslinking of casein proteins was observed.

<Test Example 8>

The effect of promoting protein crosslinking by using tyrosinase (TyrA) in combination with protein glutaminase was examined by a method using casein (Merck Millipore). The protein in an amount of 5% by weight (final concentration), a 50 mM (final concentration) potassium sodium phosphate buffer solution (pH 7.0), tyrosinase (product name: Tyrosinase from mushroom, manufactured by Merck KGaA) and protein glutaminase (product name: Protein Glutaminase "Amano" 500, manufactured by Amano Enzyme Inc.) were mixed, and then the mixture was reacted at 40°C for 24 hours with being shaken at 160 rpm. After completion of the reaction, a part of the reaction liquid was fractionated and subjected to 2 to 25% polyacrylamide electrophoresis, and an increase in the molecular weight of the substrate protein was observed to determine crosslinking and a crosslinking promotion effect relative to the case where the same concentration of tyrosinase was used alone. The enzyme addition amount was 100 U of tyrosinase (final concentration) and 500 mU of protein glutaminase (final concentration) per 1 mg of the substrate protein. Further, the same reaction was also performed under the conditions where both the enzyme addition amounts (tyrosinase and protein glutaminase) were reduced.

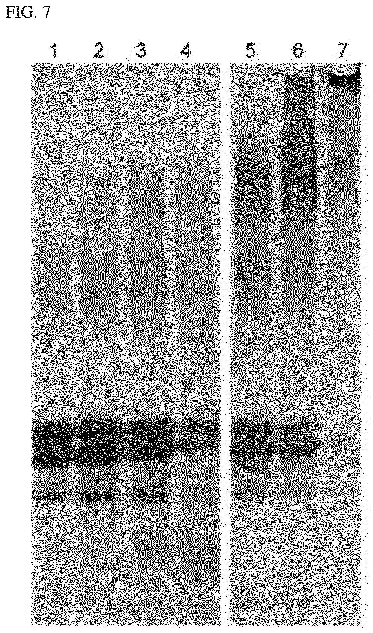

The results are shown in Fig. 7 and Table 8.

[Table 8]

| Lane | Added enzyme | Crosslinking | Crosslink promotion effect |
|---|---|---|---|
| 1 | No added enzyme | No | |
| 2 | TyrA (1/100 amount) | No | |
| 3 | TyrA (1/10 amount) | No | |
| 4 | TyrA | No | |
| 5 | TyrA + PG (1/100 amount) | Yes | Yes |
| 6 | TyrA + PG (1/10 amount) | Yes | Yes |
| 7 | TyrA + PG | Yes | Yes |

As shown in Fig. 7, as compared with the case of using tyrosinase alone, the efficiency of crosslinking polymerization of the substrate protein was improved in the case where tyrosinase and protein glutaminase were used in combination, and at any enzyme concentration, a band of the polymerized protein was observed to be denser in the upper part of each lane (lanes 5 and 6), and/or a band of the polymerized protein was newly confirmed in the topmost part of each lane (lanes 6 and 7), and crosslinking polymerization was observed. That is, by using tyrosinase in combination with protein glutaminase , the effect of promoting crosslinking of casein proteins was observed.

<Test Example 9>

(Confirmation of Viscosity Improving Effect of Casein Solution and Wheat Gluten Solution)

A mixture containing a 5% (w/v) casein solution or wheat gluten solution and protein glutaminase (product name: Protein Glutaminase "Amano" 500, manufactured by Amano Enzyme Inc.) at 100 mU/mL, laccase (product name: Laccase Y120, manufactured by Amano Enzyme Inc.) at 50 U/mL, and a 100 mM phosphate buffer solution (pH 7.0) was treated at 40°C, and the viscosity at each time was measured by EMS-1000 (Kyoto Electronics Manufacturing Co., Ltd., Tokyo, Japan). The shear rate at the time of measurement was 200 s-1, and preliminary measurement was performed for 30 seconds in order to maintain the flow state. During the measurement, a casein solution to which no enzyme was added, a casein solution treated with laccase (50 U/mL), and a casein solution treated with protein glutaminase (100 mU/mL) were also subjected to measurement as controls.

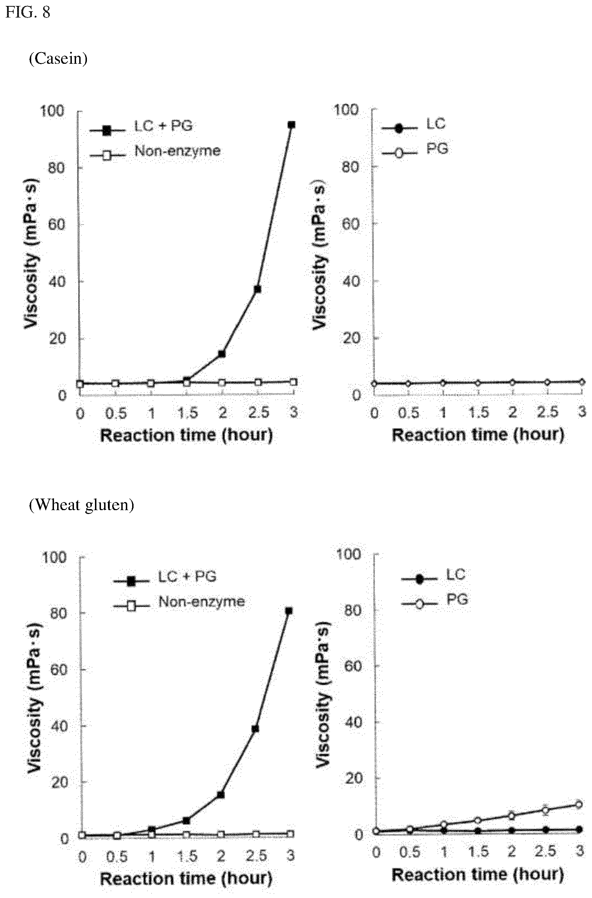

The results are shown in Fig. 8. The viscosities of the casein (or gluten) solution to which no enzyme was added, the casein (or gluten) solution to which laccase was added alone, and the casein (or gluten) solution to which protein glutaminase was added alone did not change during the measurement. In contrast, in the casein solution containing the combination of laccase and protein glutaminase, the concentration increased in a reaction time-dependent manner.

Further, also in the electrophoretic images, when laccase and protein glutaminase were used in combination, the efficiency of crosslinking polymerization of the substrate protein was improved as compared with the case where laccase was used alone, and at any enzyme concentration, the presence of a protein polymerized to greater than or equal to 200 kDa was newly confirmed, and crosslinking polymerization was observed.

That is, by using laccase in combination with protein glutaminase, the effect of promoting crosslinking of casein proteins and wheat gluten proteins was observed.

(Study on Gelling of Casein Solution)

<Test Example 10>

A 5% (w/v) casein solution and a mixture containing protein glutaminase (product name: Protein Glutaminase "Amano" 500, manufactured by Amano Enzyme Inc.) at 100 mU/mL, laccase (product name: Laccase Y120, manufactured by Amano Enzyme Inc.) at 50 U/mL, and a 100 mM phosphate buffer solution (pH 7.0) were added to a test tube and treated at 40°C for 24 hours, and the test tube was then tilted to confirm whether the solution was gelled and the crosslinking promotion effect relative to the case where laccase was used alone.

The results are shown in Table 9.

[Table 9]

| Lane | Added enzyme | Gelling | Crosslink promotion effect |
|---|---|---|---|
| 1 | No added enzyme | No | |
| 2 | LC | No | |
| 3 | PG | No | |
| 4 | PG + LC | Yes | Yes |

As shown in Table 9, the casein solution to which no enzyme was added, the casein solution to which laccase alone was added, and the casein solution to which protein glutaminase alone was added had no change in properties. In contrast, only the casein solution containing the combination of laccase and protein glutaminase gelled. That is, by using laccase in combination with protein glutaminase, the effect of promoting crosslinking of casein proteins was observed.

(Study on Gelling of Milk)

<Test Example 11 >

Commercially available milk and a mixture containing protein glutaminase (product name: Protein Glutaminase "Amano" 500, manufactured by Amano Enzyme Inc.) at 100 mU/mL, laccase (product name: Laccase Y120, manufactured by Amano Enzyme Inc.) at 50 U/mL, and a 100 mM phosphate buffer solution (pH 7.0) were added to a test tube and treated at 40°C for 24 hours, and the test tube was then tilted to confirm whether the solution was gelled and the crosslinking promotion effect relative to the case where laccase was used alone.
The results are shown in Table 10.

[Table 10]

| Lane | Added enzyme | Gelling | Crosslink promotion effect |
|---|---|---|---|
| 1 | No added enzyme | No | |
| 2 | LC | No | |
| 3 | PG | No | |
| 4 | PG + LC | Yes | Yes |

As shown in Table 10, the milk to which no enzyme was added, the milk to which laccase alone was added, and the milk to which protein glutaminase alone was added had no change in properties. In contrast, only the milk containing a combination of laccase and protein glutaminase gelled. That is, by using laccase in combination with protein glutaminase , the effect of promoting crosslinking of milk proteins was observed.

(Study on Gelling of Wheat Gluten)

<Test Example 12>

A 5% (w/v) wheat gluten solution and a mixture containing protein glutaminase (product name: Protein Glutaminase "Amano" 500, manufactured by Amano Enzyme Inc.) at 100 mU/mL, laccase (product name: Laccase Y120, manufactured by Amano Enzyme Inc.) at 50 U/mL, and a 100 mM phosphate buffer solution (pH 7.0) were added to a test tube and treated at 40°C for 24 hours, and the test tube was then tilted to confirm whether the solution was gelled and the crosslinking promotion effect relative to the case where laccase was used alone.
The results are shown in Table 11.

[Table 11]

| Lane | Added enzyme | Gelling | Crosslink promotion effect |
|---|---|---|---|
| 1 | No added enzyme | No | |

(continued)

| Lane | Added enzyme | Gelling | Crosslink promotion effect |
|------|--------------|---------|----------------------------|
| 2 | LC alone | No | |
| 3 | PG alone | No | |
| 4 | PG + LC | Yes | Yes |

As shown in Table 11, the wheat gluten solution to which no enzyme was added, the wheat gluten solution to which laccase alone was added, and the wheat gluten solution to which protein glutaminase alone was added had no change in properties. In contrast, only the wheat gluten solution containing the combination of laccase and protein glutaminase gelled. That is, by using laccase in combination with protein glutaminase , the effect of promoting crosslinking of wheat gluten proteins was observed.

INDUSTRIAL APPLICABILITY

According to the protein crosslinking method by enzymes of the present invention, the crosslinking reaction of proteins by an oxidoreductase, which has been difficult to put into practical use due to its low reactivity, can be greatly promoted. The crosslinked, polymerized or gelled protein material produced by the present invention is used in the food processing fields such as ground fish meat, steamed fish paste, fish/meat sausages, bean curd, noodles, confectionery/breadmaking, food adhesives, sheet-like meat, yogurt, jelly, cheese, and meat alternatives/dairy alternatives derived from plant raw materials (cheese alternatives and fermented dairy alternative products). Furthermore, as a novel protein-derived material, the protein material is also assumed to be used in a wide range of industries including cosmetics, medical supplies, microcapsule materials, and carriers such as immobilized enzymes. Since the reaction mechanism of crosslinking by an oxidoreductase is considered to be different from that in the case of transglutaminase frequently used for crosslinking of proteins, the use and application of the present invention to the production of protein polymerized substances or gel-like substances having new quality can also be expected.

The present invention is not limited to the description of the embodiments and examples of the invention. Various modifications that can be easily conceived by those skilled in the art without departing from the scope of the claims are also included in the present invention. The entire contents of the articles, published patent publications, patent publications, and the like specified in this specification are incorporated herein by reference.

**Claims**

1. A protein crosslinking method **characterized by** allowing an oxidoreductase and a protein deamidase to act on a protein.

2. The protein crosslinking method according to claim 1, wherein the oxidoreductase is multicopper oxidase.

3. The protein crosslinking method according to claim 2, wherein the multicopper oxidase is laccase and/or bilirubin oxidase.

4. The protein crosslinking method according to claim 2, wherein the multicopper oxidase is laccase.

5. The protein crosslinking method according to any one of claims 1 to 4, wherein the protein deamidase is an enzyme that acts on a glutamine residue in a protein.

6. The protein crosslinking method according to claim 5, wherein the protein deamidase is protein glutaminase.

7. A protein improving agent comprising:

   an oxidoreductase; and
   a protein deamidase.

8. The protein improving agent according to claim 7, wherein the oxidoreductase is multicopper oxidase.

9. The protein improving agent according to claim 8, wherein the multicopper oxidase is laccase and/or bilirubin oxidase.

10. The protein improving agent according to claim 8, wherein the multicopper oxidase is laccase.

11. The protein improving agent according to any one of claims 7 to 10, wherein the protein deamidase is an enzyme that acts on a glutamine residue in a protein.

12. The protein improving agent according to claim 11, wherein the protein deamidase is protein glutaminase.

13. A method for producing a crosslinked protein, the method comprising the steps of:

(1) treating a protein with a protein deamidase; and
(2) treating the protein subjected to protein deamidation with an oxidoreductase.

14. A method for producing a crosslinked protein, the method comprising the steps of:

(1) preparing a protein treated with a protein deamidase; and
(2) treating the prepared protein with an oxidoreductase.

15. A method for producing a crosslinked protein, the method comprising simultaneously treating a protein with an oxidoreductase and a protein deamidase.

16. A method for producing a food or a pharmaceutical product, the method comprising the steps of:

(1) preparing a food raw material or pharmaceutical raw material containing a protein and treated with a protein deamidase; and
(2) treating the prepared food raw material or pharmaceutical raw material with an oxidoreductase.

17. A method for producing a food or a pharmaceutical product, the method comprising a step of simultaneously treating a food raw material or a pharmaceutical raw material containing a protein with an oxidoreductase and a protein deamidase.

FIG. 1

FIG. 2

FIG. 3

FIG. 4

FIG. 5

FIG. 6

FIG. 7

FIG. 8

(Casein)

(Wheat gluten)

## INTERNATIONAL SEARCH REPORT

| | International application No. |
|---|---|
| | PCT/JP2021/010764 |

### A. CLASSIFICATION OF SUBJECT MATTER
A23L 5/00(2016.01)i; C12N 9/02(2006.01)i; C12N 9/78(2006.01)i; C12P 21/00(2006.01)i
FI: C12P21/00; C12N9/02; C12N9/78; A23L5/00 M; A23L5/00 J

According to International Patent Classification (IPC) or to both national classification and IPC

### B. FIELDS SEARCHED

Minimum documentation searched (classification system followed by classification symbols)
A23L5/00; C12N9/00-9/99; C12P21/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); PubMed

### C. DOCUMENTS CONSIDERED TO BE RELEVANT

| Category* | Citation of document, with indication, where appropriate, of the relevant passages | Relevant to claim No. |
|---|---|---|
| X | JUVONEN, H. et al., "Film Formation and Surface Properties of Enzymatically Crosslinked Casein Films", Journal of Applied Polymer Science, 2011, vol. 119, pp. 2205-2213, fig. 2, D, page 2206, left column, lines 10-34 | 1, 5, 15 |
| A | entire text | 2-4, 6-14, 16-17 |

☒ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 May 2021 (20.05.2021) | 01 June 2021 (01.06.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

23

**INTERNATIONAL SEARCH REPORT**

International application No.

PCT/JP2021/010764

C (Continuation).   DOCUMENTS CONSIDERED TO BE RELEVANT

| Category* | Citation of document, with indication, where appropriate, of the relevant passages | Relevant to claim No. |
|---|---|---|
| X | 山崎 勝利，トランスグルタミナーゼによる麺の経時変化時の劣化耐性付与，月刊フードケミカル，2003, vol. 19, pp. 44-49, page 44, right column, line 2 to page 45, left column, line 1 from the bottom, page 45, right column, line 1 to page 49, left column, line 1 from the bottom | 1-5, 7, 11, 15, 17 |
| Y | page 44, right column, line 2 to page 45, left column, line 1 from the bottom, page 45, right column, line 1 to page 49, left column, line 1 from the bottom | 1-5, 7-11, 15, 17 |
| A | entire text, non-official translation (YAMAZAKI, Katsutoshi, "Conferring deterioration resistance on change over time of noodle by transglutaminase", Food chemicals) | 6, 12-14, 16 |
| Y | JP 11-276162 A (AMANO PHARMACEUT CO., LTD.) 12 October 1999 (1999-10-12) claims, paragraphs [0002]-[0007] entire text | 1-5, 7, 11, 15, 17 |
| A | | 6, 12-14, 16 |
| X | JP 2000-060431 A (AJINOMOTO CO., INC.) 29 February 2000 (2000-02-29) claims, examples entire text | 1, 5, 7, 11, 13-17 |
| A | | 2-4, 6, 8-10, 12 |
| A | Hou, J. J. et al., "Preparation of double-network tofu with mechanical and sensory toughness", International Journal of Food Science and Technology, 2016, vol. 51, pp. 962-969, entire text | 1-17 |

Form PCT/ISA/210 (continuation of second sheet) (January 2015)

**INTERNATIONAL SEARCH REPORT**
Information on patent family members

International application No.

PCT/JP2021/010764

| Patent Documents referred in the Report | Publication Date | Patent Family | Publication Date |
|---|---|---|---|
| JP 11-276162 A | 12 Oct. 1999 | US 2002/0009770 A1 claims, paragraphs [0002]-[0007] | |
| JP 2000-060431 A | 29 Feb. 2000 | EP 963704 A2 claims, examples | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0665280 A
- JP H11276162 A
- WO 2015133590 A

**Non-patent literature cited in the description**

- **MATHEIS ; WHITAKER.** *J. Food Biochemistry,* 1987, vol. 11, 309-327
- *Nippon Nogeikagaku Kaishi,* vol. 69 (10), 1301-1308
- *EurJBiochem,* 2001, vol. 268 (5), 1410
- **S YAMAGUCHI 1 ; DJ JEENES ; DBARCHER.** *Front Microbiol,* 2018, vol. 9, 1975
- Molecular Approaches to Improving Food Quality and Safety. Van Nostrand Reinhold, 1992, 37